# EUROPÄISCHE PATENTSCHRIFT

(11) **EP 2 173 704 B1**
(45) Veröffentlichungstag und Bekanntmachung des Hinweises auf die Patenterteilung: **11.09.2013**
(21) Anmeldenummer: 08786184.5
(22) Anmeldetag: 16.07.2008
(51) Int. Cl.: C07C 253/30, C07C 255/07, C11B 9/00, C11D 3/50, A61L 9/01, A61Q 13/00, A61K 8/40

(54) **VERFAHREN ZUR HERSTELLUNG VON ETHYLGERANONITRIL**
PROCESS FOR THE PREPARATION OF ETHYLGERANONITRILE
PROCÉDÉ DE PRODUCTION D'ÉTHYLGÉRANONITRILE

(30) Priorität: 26.07.2007 EP 07113268
(43) Veröffentlichungstag der Anmeldung: 14.04.2010
(73) Patentinhaber: BASF SE, 67056 Ludwigshafen (DE)
(72) Erfinder: WEIS, Martine, 68165 Mannheim (DE); BRUNNER, Bernhard, 69120 Heidelberg (DE); EBEL, Klaus, 68623 Lampertheim (DE); KRAUSE, Wolfgang, 68782 Brühl-Rohrhof (DE)
(86) Internationale Anmeldenummer: PCT/EP2008/059290
(87) Internationale Veröffentlichungsnummer: WO 2009/013199

(56) Entgegenhaltungen:
- EP-A- 0 074 253
- WO-A-02/081614
- US-A- 3 655 722
- US-A- 3 960 923
- "3,7-Dimethyl-2,6-nonadienenitrile" FOOD AND CHEMICAL TOXICOLOGY, XX, XX, Bd. 30, 1. Januar 1992 (1992-01-01), Seite 27, XP023810941 ISSN: 0278-6915 [gefunden am 1992-01-01]
- DATABASE WPI Week 200570 Thomson Scientific, London, GB; AN 2005-678472 XP002505320 & JP 2005 239664 A (KAO CORP) 8. September 2005 (2005-09-08)
- DATABASE CA [Online] CHEMICAL ABSTRACTS SERVICE, COLUMBUS, OHIO, US; 12. Mai 1984 (1984-05-12), WATANABE, SHOJI ET AL: "Reaction of 6-methyl-5-hepten-2-on with malononitrile" XP002505319 gefunden im STN Database accession no. 90:168745 & YUKAGAKU , 27(12), 863-5 CODEN: YKGKAM; ISSN: 0513-398X, 1978,

## Beschreibung

Die vorliegende Erfindung betrifft Gemische umfassend 3,7-Dimethyl-2,6-nonadiennitril und mindestens eine Verbindung ausgewählt aus der Gruppe der Verbindungen 3,7-Dimethyl-3,6-nonadiennitril und 3-Methylen-7-methyl-6-nonennitril wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt sowie ein Verfahren zur Herstellung solcher Gemische.

Aliphatische Nitrile stellen wertvolle Aromachemikalien dar, die sich insbesondere auf Grund der chemischen Stabilität der Nitril-Gruppe zur Anwendung unter nicht-neutralen Bedingungen, beispielsweise unter basischen oder stark sauren Bedingungen eignen. Demzufolge besteht ein steter Bedarf an neuen aliphatischen Nitrilen oder Mischungen derselben mit interessanten Geruchseigenschaften sowie an Verfahren zur wohlfeilen Herstellung derartiger Stoffe oder Stoffgemische.

Eines der auf Grund ihrer speziellen Riechstoffeigenschaften sehr gefragten aliphatischen Nitrile stellt das Geranonitril (3,7-Dimethyl-2,6-octadiennitril) dar, das ein sehr intensiv zitrusartiges Geruchsprofil mit bitteren und cuminischen sowie frischheuartigen Facetten aufweist. Eine Alternative dazu stellt das auch als Lemonile® vertriebene 3,7-Dimethyl-2,6-nonadiennitril (Ethylgeranonitril) das, welches in wesentlichen Aspekten gleichwertige bzw. sogar vorteilhafte geruchliche Facetten aufweist. Dieses Produkt vermittelt eine natürlichere Ausstrahlung bei nachhaltigerer Wirkung und reduzierten bitteren Facetten und ist demgemäss als Substitut für Geranonitril sehr gut geeignet.

Da Geranonitril auf Grund neuerer toxikologischer Erkenntnisse als sogenannter cmr2-Stoff beurteilt wird, ist damit zu rechnen, dass die industrielle Herstellung in Zukunft einzustellen ist.

Verfahren zur Herstellung α,β-ungesättigter Nitrile sind dem Fachmann bekannt. So offenbart die DE 21 35 666 ein Verfahren zur Herstellung von Cycloalkylidenacetonitrilen durch Umsetzung von Cycloalkanonen mit Acetonitril in flüssiger Phase in Gegenwart katalytischer Mengen von Alkali- oder Erdalkalialkoholaten.

Die US 3,960,923 betrifft ein Verfahren zur Herstellung α,β-ungesättigter Nitrile durch Umsetzung eines Ketons mit Acetonitril in Gegenwart einer starken Base. Als geeignete Ketone werden aliphatische Ketone, darunter Methylheptenon genannt.

Die EP 0 074 253 offenbart spezielle gesättigte, α- oder β-substituierte aliphatische Nitrile, deren Verwendung als Riechstoffe sowie Verfahren zu ihrer Herstellung. So wird die Umsetzung von Methylalkylketonen mit Tosylmethylisocyanid mit Kalium-tert.-butoxid in Diglyme beschrieben. Im Rahmen eines weiteren Verfahrens werden Methylalkylketone mit Cyanessigsäure in Gegenwart von Ammoniumacetat umgesetzt.

Die US 4,361,702 betrifft ein Verfahren zur Herstellung von Acetalen des 3-Formyl-2-butennitrils durch Umsetzung des entsprechend acetalisierten Methylketons mit Acetonitril in Gegenwart einer starken Base.

S. DiBiase et al. beschreiben in Org. Synth. Coll. Vol. 7, 1990, 108 -112 bzw. Org. Synth. Vol. 62, 1984, 179 - 185 die Umsetzung von Cyclohexanon oder Benzaldehyd mit Acetonitril in Gegenwart von Kaliumhydroxid zu den entsprechenden α,β-ungesättigten Nitrilen.

S. DiBiase et al. beschreiben darüber hinaus in J. Org. Chem., Vol. 44, 1979, 4640 - 4649 die Herstellung von α,β-ungesättigten Nitrilen durch Umsetzung von Carbonylverbindungen mit Acetonitril in Gegenwart von Basen und gegebenenfalls Kronenethern.

A. Valla et al. beschreiben in Synthetic Communications, Vol. 33, No. 7, 2003, pp. 1195 - 1201, die Umsetzung von 6-Methyl-5-hepten-2-on mit Acetonitril in Gegenwart von Kaliumhydroxid unter Erhalt von Geranonitril in einer Ausbeute von 65%.

Die US 3,655,722 betrifft 7-Methyl-3-methylen-6-octennitril sowie 3,7-Dimethyl-2,6-octadiennitril (Geranonitril) sowie ein Gemisch der cis- und trans-Isomere des Geranonitrils und weitere Doppelbindungsisomere von 3,7-Dimethyloctadiennitril. Die Verbindungen werden durch Umsetzung von 2-Methyl-2-hepten-6-on mit Cyanessigsäure in Gegenwart von Triethanolamin oder Cyclohexylamin erhalten.

Food and Chemical Toxicology, Bd. 30, 1.1.1992, S 27 offenbart ein Verfahren zur Synthese von 3,7-Dimethyl-2,6-nonadiennitiril.

JP 2005 239664A (Kao Corp.) beschreibt ein Haarschutzmittel enthaltend Citronellylnitril und/oder 3,7-Dimethyl-2(3),6-nonadiennitril.

WO 02/081614 offenbart eine Duftkomposition enthaltend 3,7-Dimethyl-6-octennitril und 3,7-Dimethyl-2,6-nonadiennitril und deren Verwendung in Waschmitteln.

Chem Abstracts Database Acc No 90:168745 offenbart die Kondensation von 6-Methyl-5-hepten-2-on mit Cyanoessigsäureethylester.

Die Aufgabe der vorliegenden Erfindung bestand in der Bereitstellung eines Verfahrens zur Herstellung von Gemischen enthaltend Ethylgeranonitril (3,7-Dimethyl-2,6-nonadiennitril). Das Verfahren soll die Herstellung von Isomerengemischen desselben ermöglichen und in verfahrenstechnisch möglichst einfacher Weise im technischen Maßstab durchführbar sein. Es soll, ausgehend von wohlfeilen, gut zugängliche Ausgangsstoffen das gewünschte Produkt in möglichst hoher Ausbeute und unter Vermeidung unerwünschter Nebenprodukte liefern. Darüber hinaus soll das Produkt in einer Form, d.h. in Form von Isomerengemischen, anfallen, in der es sich als Substitut für Geranonitril eignet. Insbesondere soll es ein mit Geranonitril vergleichbares, möglichst ebenbürtiges und bevorzugt angenehmeres bzw. attraktiveres Geruchsprofil aufweisen und eine leichte Austauschbarkeit des Geranonitrils gewährleisten.

Die Aufgabe wurde gelöst durch die Bereitstellung eines Verfahrens zur Herstellung eines Gemisches umfassend 3,7-Dimethyl-2,6-nonadiennitril und mindestens eine Verbindung, ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt, umfassend die Schritte
a. Umsetzung eines Nitrils der Formel (II) 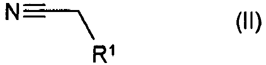 in dem der Rest
   R¹ Wasserstoff bedeutet,
   mit einer Base als Aktivierungsreagenz unter Erhalt des Nitrils der Formel (II) in deprotonierter Form und
b. Umsetzung des Nitrils der Formel (II) in deprotonierter Form mit 6-Methyl-5-octen-2-on unter Erhalt von 3,7-Dimethyl-2,6-nonadiennitril.

Das erfindungsgemäße Verfahren eignet sich zur Herstellung von 3,7-Dimethyl-2,6-nonadiennitril der Formel (I) 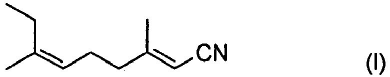 wobei die Verbindung üblicherweise in Form eines Gemisches von Konfigurationsisomeren (d.h. E/Z-Isomeren) bezüglich der beiden ethylenische Doppelbindungen und in Form von Gemischen mit Konstitutionsisomeren bezüglich der Doppelbindung in α,β-bzw. β,γ-Position zur Nitrilgruppe anfällt.

Als mögliche Konfigurationsisomere, die neben dem Isomeren der Formel (I) üblicherweise erhalten werden, seien die Verbindungen (Ia) bis (Ic) 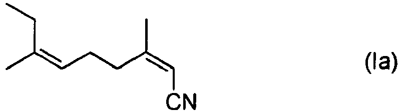 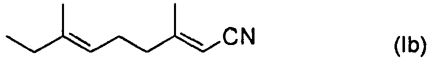 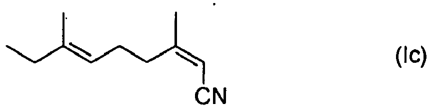 genannt, die sich lediglich bezüglich der E/Z-Konfiguration der ethylenischen Doppelbindungen voneinander unterscheiden. 3,7-Dimethyl-2,6-nonadiennitril, d.h. die Verbindungen der Formeln (I) sowie (Ia) bis (Ic) weist bzw. weisen eine ethylenische Doppelbindung in α,β-Position zur Nitrilgruppe auf und werden im Rahmen der vorliegenden Erfindung auch als konjugierte Isomere bezeichnet.

Als Konstitutionsisomere, die neben dem als Hauptprodukt erhaltenen 3,7-Dimethyl-2,6-nonadiennitril erhalten werden, seien die Verbindungen 3,7-Dimethyl-3,6-nonadiennitril und 3-Methylen-7-methyl-6-nonennitril genannt. 3,7-Dimethyl-3,6-nonadiennitril und 3-Methylen-7-methyl-6-nonennitril können ebenfalls in Form von Gemischen der Konfigurations- d.h. E/Z-Isomere erhalten werden. So wird 3-Methylen-7-methyl-6-nonennitril üblicherweise in Form eines Gemisches der beiden Isomere der Formeln (V) bzw. (Va) 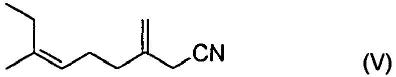 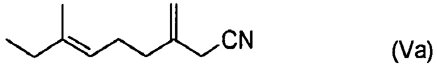 erhalten. 3,7-Dimethyl-3,6-nonadiennitril wird üblicherweise in Form von Gemischen aller vier Konfigurationsisomere der Formeln (VI) und (VIa) bis (VIc) 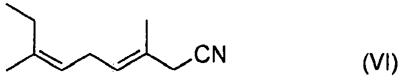 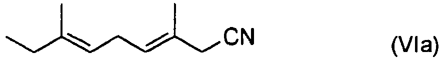 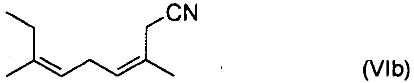 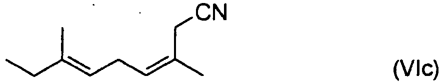 erhalten. 3,7-Dimethyl-3,6-nonadiennitril und 3-Methylen-7-methyl-6-nonennitril weisen eine ethylenische Doppelbindung in β,γ-Position zur Nitrilgrupe auf und werden im Rahmen der vorliegenden Erfindung zusammen auch als nicht-konjugierte Isomere bezeichnet.

Die erfindungsgemäß zugänglichen Gemische enthalten üblicherweise 3,7-Dimethyl-2,6-nonadiennitril (d.h. die Verbindung der Formel (I) sowie ihre Isomere der Formel (Ia) bis (Ic)) als Hauptkomponente in einem Anteil von in der Regel etwa 40 bis etwa 100 Gew.-%, bevorzugt 50 bis 100 Gew.-% und besonders bevorzugt 60 bis 100 Gew.-%, bezogen auf das erhaltene Isomerengemisch der Verbindungen der Formeln (I), (V) und (VI) und deren Isomere.

Die genannten nicht-konjugierten Isomere, d.h. 3,7-Dimethyl-3,6-nonadiennitril und 3-Methylen-7-methyl-6-nonennitril, werden üblicherweise, je nach Wahl der Reaktionsbedingungen, jeweils in geringerem Ausmaß gebildet.

Als Ausgangsstoff zur Durchführung des erfindungsgemäßen Verfahrens dient ein Nitril der Formel (II)

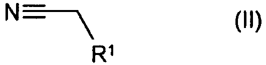

in der Rest R¹ Wasserstoff bedeutet. Demnach dient Acetonitril als Ausgangsstoff der Formel (II).

Das gewählte Nitril der Formel (II) wird gemäß Schritt a) des erfindungsgemäßen Verfahrens mit einer Base als Aktivierungsreagenz unter Erhalt des Nitrils der Formel (II) in deprotonierter Form umgesetzt. Unter dem Begriff Aktivierungsreagenz sind dabei solche Reagenzien zu verstehen, die das eingesetzte Acetonitril, in α-Position, d.h. am Kohlenstoffatom in Nachbarschaft zur Nitrilgruppe deprotonieren können, bevorzugt solche Reagenzien, die einen pKs-Wert von 4 oder darüber aufweisen, d.h. schwache Basen (Brönstedt-Basen). Man erhält das Nitril der Formel (II) in deprotonierter Form wie beispielsweise durch Formel (IIb) dargestellt, wobei als Aktivierungsreagenz eine Base eingesetzt wurde und wobei M⁺ das Gegenion der eingesetzten Base darstellt:

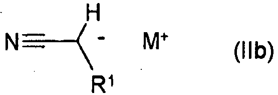

Im Falle des erfindungsgemäßen Einsatzes von Alkalimetallhydroxiden wie Natriumhydroxid (NaOH) oder Kaliumhydroxid (KOH) als Aktivierungsreagenzien bzw. Basen wären unter den Gegenionen M⁺ der vorstehend genannten Formeln (IIa) und (IIb) dementsprechend Na⁺ oder K⁺ zu verstehen.

Im Rahmen des erfindungsgemäßen Verfahrens setzt man als Aktivierungsreagenz eine Base oder ein Gemisch verschiedener Basen ein. Als diesbezüglich geeignete Basen seien solche genannt, die einen pKs-Wert von etwa 5 bis etwa 50, bevorzugt etwa 9 bis etwa 25 aufweisen, wobei der Begriff pKs- Wert wie in Organikum 21. Auflage, Weinheim, Wiley-VCH, 2001, S.156 - 159 definiert ist. Beispielhaft seien als erfindungsgemäß bevorzugt einzusetzende Basen genannt: Alkali- oder Erdalkalihydroxide wie beispielsweise: LiOH, NaOH, KOH, Ba(OH)₂; Ammoniumhydroxide; Alkali- oder Erdalkalialkoholate von kurzkettigen Alkoholen, vorzugsweise solche mit 1 bis 6, bevorzugt 1 bis 4 Kohlenstoffatome wie beispielsweise Natriummethanolat, Natriumethanolat, Kaliummethanolat, Kaliumethanolat, Natriumpropylat, Kaliumpropylat, Natriumisopropylat, Kaliumisopropylat, Natriumbutylat, Kaliumbutylat, Natriumisobutylat, Kaliumisobutylat.

Außerdem können generell zur Umsetzung von Acetonitril auch Metallorganyle wie beispielsweise Butyllithium oder Amide wie beispielsweise Lithium- oder Natriumamid eingesetzt werden.

Im Fall des erfindungsgemäß eingesetzten Acetonitrils setzt man als Aktivierungsreagenz vorteilhaft Basen mit einem pKs-Wert von 12 bis 50, bevorzugt mit einem pKs-Wert von 15 bis 45 ein.

Das gewählte Aktivierungsreagenz kann stöchiometrisch oder katalytisch in einem Verhältnis von 0,01 bis 10 mol/mol, bevorzugt 0,1 bis 5 mol/mol, besonders bevorzugt 0,5 bis 1.5 mol/mol Carbonylverbindung (6-Methyl-5-octen-2-on) eingesetzt werden. Zusätzlich kann es, je nach Art des verwendeten Aktivierungsreagenzes, von Vorteil sein, Additive wie beispielsweise Kronenether, Phasentransferkatalysatoren oder Säuren wie zum Beispiel Eisessig zu dem Reaktionsgemisch hinzuzufügen.

Die genannten Aktivierungsreagenzien können als solche in fester oder gegebenenfalls flüssiger Form oder in Form von Lösungen in geeigneten Lösungsmitteln oder Gemischen derselben eingesetzt werden. Die Umsetzung gemäß Schritt a) des erfindungsgemäßen Verfahrens wird üblicherweise so vorgenommen, dass man das gewählte Nitril der Formel (II) als solches oder in Form einer Lösung in einem geeigneten, unter den Reaktionsbedingungen inerten Lösungsmittel wie beispielsweise Benzol, Toluol, Xylole, Pentan, Hexan, Cyclohexan, Heptan, Diethylether, Petrolether, 1,4-Dioxan, Tetrahydrofuran, N,N-Dimethylformamid (DMF), Methanol, Ethanol, Propanol, Butanol, Methylenchlorid oder Chloroform vorlegt und das gewählte Aktivierungsreagenz, d.h. bevorzugt die gewählte Base, zugibt. In einer erfindungsgemäß bevorzugten Ausführungsform führt man die Reaktion in Acetonitril als Lösungsmittel durch. Dieses kann dann auch als Ausgangsstoff der Formel (II) dienen. Ein weiterer Vorteil von Acetonitril als Lösungsmittel ist seine Fähigkeit, durch Bildung von Acetamid das während der Reaktion entstehende Wasser aus dem Gemisch zu entziehen. Andere Abfangmittel wie beispielsweise Molekularsieb können ebenfalls zugesetzt werden.

Gemäß Schritt b) des erfindungsgemäßen Verfahrens setzt man das wie vorstehend beschrieben erhaltene Nitril der Formel (II) in deprotonierter Form mit 6-Methyl-5-octen-2-on, das im Rahmen der vorliegenden Erfindung auch als Ethylheptenon bezeichnet wird und in Form eines Gemisches der E/Z-Isomeren der Formel (III) und (IIIa) 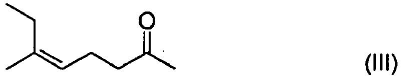 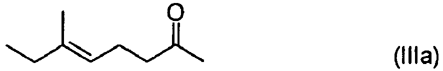 eingesetzt wird, um.

Die erfindungsgemäßen Reaktionsschritte a) und b) können separat nacheinander oder in einem Reaktionsgemisch aus dem Nitril der Formel (II), dem gewählten Aktivierungsreagenz sowie dem Ethylheptenon durchgeführt werden.

Die als Ausgangsstoffe im Rahmen des erfindungsgemäßen Verfahrens einzusetzenden Nitril der Formel (II), sowie Ethylheptenon werden üblicherweise in etwa äquimolarem Verhältnis miteinander umgesetzt. Acetonitril kann auch als Lösungsmittel in großem Überschuss eingesetzt werden kann.

Die Menge an zugesetzter Base kann über einen breiten Bereich variiert werden, da die Reaktion in Abhängigkeit von der Stärke der eingesetzten Base sowie in Abhängigkeit von der Wahl des Ausgangsstoffes der Formel (II) in katalytischer oder stöchiometrischer Form ausgeführt werden kann.

Im Rahmen einer bevorzugten Ausführungsform führt man das erfindungsgemäße Verfahren so durch, dass man zunächst im Rahmen des erfindungsgemäßen Schrittes a) das eingesetzte Nitril der Formel (II) vollständig oder zum überwiegenden Teil in das entsprechende Anion durch Umsetzung mit einer etwa äquimolaren Menge, üblicherweise einer Menge von etwa 0,9 bis etwa 1,2 mol-Äquivalenten, bevorzugt etwa 0,95 bis etwa 1,1 mol-Äquivalenten einer geeigneten, wie vorstehend beschriebenen Base deprotoniert und anschließend gemäß Schritt b) mit Ethylheptenon umsetzt. Im Rahmen einer weiterhin bevorzugten Ausführungsform führt man das erfindungsgemäße Verfahren so durch, dass man das gemäß Schritt a) erhaltene Reaktionsgemisch vorlegt und Ethylheptenon, bevorzugt langsam, zudosiert.

Im Rahmen einer bevorzugten Ausführungsform des erfindungsgemäßen Verfahrens setzt man gemäß Schritt a) Acetonitril als ein, welches mit einer starken Base, ausgewählt aus der Gruppe der Basen umfassend die Alkali- oder Erdalkalihydroxide, Ammoniumhydroxid und Alkali- oder Erdalkalialkoholate, in einer Menge von etwa 0,95 bis 1,2 mol-Äquivalenten (bezogen auf das in Schritt b) einzusetzende Ethylheptenon) zunächst vollständig bzw. größtenteils in das entsprechende deprotonierte Acetonitril überführt wird. Dabei kann das Acetonitril auch als Lösungsmittel im (großen) Überschuss eingesetzt werden. Die Umsetzung verläuft, in Abhängigkeit von der gewählten Base und der Reaktionstemperatur, üblicherweise rasch ab und ist bei Einsatz eines Alkalialkoholats wie beispielsweise Natriummethanolat bei Raumtemperatur, bei Einsatz eines Alkalihydroxids wie beispielsweise KOH bei gegebenenfalls leicht erhöhten Temperaturen von bis zu etwa 80°C üblicherweise innerhalb einiger Minuten bis etwa 1 h weitgehend abgeschlossen.

Die so bereitete Lösung des deprotonierten Nitrils kann dann gemäß Schritt b) mit dem Ethylheptenon umgesetzt werden. Dies geschieht vorteilhaft unter langsamer Zudosierung des Ketons, um unerwünschte Nebenreaktionen so weit wie möglich zu vermeiden.

Die Umsetzung des Ethylheptenons mit dem deprotonierten Nitril der Formel (II) erfolgt ebenfalls rasch und ist bei Temperaturen von etwa 20 bis etwa 180°C üblicherweise nach etwa 1 bis etwa 24 h weitgehend abgeschlossen. Je nach Art der Reaktionsführung bzw. in Abhängigkeit vom eingesetzten Lösungsmittel kann es vorteilhaft sein, die erfindungsgemäße Umsetzung mit Ethylheptenon unter Abscheidung des bei der Reaktion in stöchiometrischer Menge entstehenden Wassers durchzuführen. Zu diesem Zweck kann es in manchen Fällen auch von Vorteil sein, ein zusätzliches, inertes Lösungsmittel wie beispielsweise Toluol oder Xylol zuzugeben, welches als Schleppmittel für das entstandene Reaktionswasser dient.

Die Umsetzung des deprotonierten Nitrils der Formel (II) mit Ethylheptenon führt im Falle der Umsetzung von Acetonitril in erfindungsgemäßer Weise direkt zum 3,7-Dimethyl-2,6-nonadiennitril bzw. zu dem Isomerengemisch umfassend 3,7-Dimethyl-2,6-nonadiennitril.

Die erfindungsgemäß erhaltenen Reaktionsgemische können nach dem Fachmann bekannten Methoden weiter aufgearbeitet werden, beispielsweise mittels extraktiver Verfahren und anschließend beispielsweise durch destillative Verfahren weiter aufgereinigt werden.

Im Rahmen einer weiterhin bevorzugten Ausführungsform unterwirft man die erfindungsgemäß erhaltenen Produktgemische, einer nachgeschalteten Isomerisierung durch Behandlung bzw. Inkontaktbringen mit einer Base, bevorzugt einer starken Base. Als im Rahmen der nachgeschalteten zusätzliche Isomerisierung geeignete Basen seien die Alkalialkoholate und -hydroxide sowie die Erdalkalialkoholate wie z.B. Natriummethylat, Natrium-ethylat, Natriumpropylat, Natriumbutylat, Kaliummethylat, Kaliumethylat und dergleichen mehr genannt. Diese können in katalytischen oder stöchiometrischen Mengen bzw. im Überschuss eingesetzt werden und zwar üblicherweise in Mengen von 0,01 mol bis 10 mol/mol, bevorzugt 0,1 bis 1 mol/mol, des Isomerengemischs. Als geeignete Lösungsmittel, die gegenüber den eingesetzten Ausgangsstoffen bzw. Reagenzien unter den Reaktionsbedingungen inert sein sollten, seien beispielhaft genannt: Benzol, Toluol, Xylole, Methanol, Ethanol, Propanol, Butanol. Als Reaktionstemperatur zur Durchführung der Nachisomerisierung sind Temperaturen im Bereich von -80°C bis 120°C, bevorzugt von 0 bis 50°C geeignet. Die Isomerisierungen verlaufen üblicherweise rasch und sind in der Regel nach Reaktionszeiten von etwa 1 bis etwa 48 h, oft nach etwa 1 bis etwa 5 h, zumindest weitgehend abgeschlossen.

Das erfindungsgemäße Verfahren erlaubt die Herstellung von 3,7-Dimethyl-2,6-nonadiennitrit (Ethylgeranonitril), das üblicherweise in Form von Gemischen der Konfigurationsisomere der Formel (I), (Ia), (Ib) und (Ic) anfällt. Neben den genannten konjugierten Isomeren umfassen die erfindungsgemäß zugänglichen Gemische auch die nicht-konjugierten, konstitutionsisomeren Verbindungen 3,7-Dimethyl-3,6-nonadiennitril und 3-Methylen-7-methyl-6-nonennitril der Formeln (V) bzw. (Va) und/oder (VI), (VIa), (VIb) und (VIc). Die vorliegende Erfindung betrifft daher auch die durch das erfindungsgemäße Verfahren erhältlichen 3,7-Dimethyl-2,6-nonadiennitril umfassenden Gemische, gemäß Anspruch 1.

Das erfindungsgemäße Verfahren eröffnet die Möglichkeit, zur Herstellung von Gemischen bzw. von Rohproduktgemischen mit einem besonders geringen Gehalt der genannten nicht-konjugierten Isomere 3,7-Dimethyl-3,6-nonadiennitril und/oder 3-Methylen-7-methyl-6-nonennitril.

Die Erfindung betrifft daher Gemische, umfassend 3,7-Dimethyl-2,6-nonadiennitril und mindestens eine Verbindung ausgewählt aus der Gruppe der Verbindungen 3,7-Dimethyl-3,6-nonadiennitril und 3-Methylen-7-Methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadien-nitril im Gemisch 0,05 bis 6 Gew.-% beträgt. Dabei sowie nachfolgend umfassen die genannten Verbindungen jeweils die möglichen E/Z-Doppelbindungsisomere bezüglich der ethylenischen Doppelbindungen.

Insbesondere bevorzugt beträgt der gemeinsame Gehalt an 3,7-Dimethyl-3,6-nonadiennitril und 3-Methylen-7-Methyl-6-nonennitril 0,75 bis 5 Gew.-%, bevorzugt 1 bis 5 Gew.-%, jeweils bezogen auf die Menge an 3,7-Dimethyl-2,6-nonadiennitril im Gemisch.

Die erfindungsgemäßen Gemische enthalten die genannten Verbindungen 3,7-Dimethyl-2,6-nonadiennitril, 3,7-Dimethyl-3,6-nonadiennitril und/oder 3-Methylen-7-Methyl-6-nonennitril in einem (auf das gesamte Gemisch bezogenen) gemeinsamen Anteil von üblicherweise 80 bis 100 Gew.-%, bevorzugt 85 oder 90 bis 99,9 Gew.-%, besonders bevorzugt 95 bis 99,5, insbesondere bevorzugt bis 99 oder bis 98 Gew.-%. Erfindungsgemäß bevorzugte Gemische bestehen im wesentlichen, d.h. zu mindestens 90 Gew.-% bevorzugt zu mindestens 95 Gew.-%, besonders bevorzugt zu mindestens 98 Gew.-% und ganz besonders bevorzugt zu mindestens 99 Gew.-% aus den Verbindungen 3,7-Dimethyl-2,6-nonadiennitril, 3,7-Dimethyl-3,6-nonadiennitril und/oder 3-Methylen-7-methyl-6-nonennitril und in geringem Ausmaß, bevorzugt in einer Menge von 0,1 bis 2, bevorzugt von 0,5 bis 1 Gew.-% aus weiteren Komponenten bzw. Verunreinigungen.

Die erfindungsgemäßen Gemische weisen einen intensiven zitronenartigen Duft mit natürlicher Ausstrahlung auf. Die erfindungsgemäßen Gemische substituieren in vorteilhafter Weise Geranonitril (3,7-Dimetyl-2,6-octadiennitril), insbesondere in den Fällen, in denen Gemische eine hohe Reinheit bezüglich der (E,Z)-2,6-konfigurierten Doppelbindungsisomere, d.h. bezüglich der konjugierten Isomere aufweisen. Die erfindungsgemäßen Gemische eignen sich daher in besonderem Maße als Alternativen bzw. Ersatzstoff für den Riechstoff Geranonitril.

Die vorliegende Erfindung betrifft daher auch die Verwendung der Gemische umfassend 3,7-Dimethyl-2,6-nonadiennitril und mindestens eine Verbindung ausgewählt aus der Gruppe der Verbindungen 3,7-Dimethyl-3,6-nonadiennitril und 3-Methylen-7-Methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt, als Riechstoffe.

Insbesondere eignen sich die erfindungsgemäßen Gemische als Riechstoffe zur Erzeugung eines Zitrusgeruchs, wie er vorstehend charakterisiert ist. Die vorliegende Erfindung betrifft demnach in einem weiteren Aspekt die Verwendung der erfindungsgemäßen Gemische als Riechstoffe zur Erzeugung eines Zitronenduftes.

Die erfindungsgemäß zugänglichen, wie vorstehend beschriebenen Gemische können zur Aromatisierung derselben in Verbrauchs- oder Gebrauchsgüter eingearbeitet bzw. auf solche aufgebracht werden und ihnen dadurch einen angenehmen zitronenartigen Duft verleihen. Beispielhaft seien als mit den erfindungsgemäßen Gemischen aromatisierbare Verbrauchs- oder Gebrauchsgüter genannt: Reinigungsmittel wie beispielsweise Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Waschmittel, Weichspüler, Wäschebehandlungsmitte, Texfilbehandlungsmittel wie beispielsweise Bügelhilfsmittel, daneben Bleichen bzw. Bleichlaugen, Toilettensteine, Entkalkungsmittel, Raumbedufter (Air Care), Duftstoffkompositionen, beispielsweise für die Feinparfümerie, kosmetische Mittel, aber auch Düngemittel, Baustoffe, Schimmelentfemer, Desinfektionsmittel, Produkte für die Auto- bzw. Fahrzeugpflege und dergleichen mehr.

Aufgrund ihrer chemischen Ähnlichkeit zum Riechstoff Geranonitril eignen sich die erfindungsgemäßen Gemische insbesondere für die üblichen Anwendungen des Geranonitrils, das aufgrund seiner chemischen Stabilität mit Vorteil auch in aggressiven Medien wie Säuren oder Laugen oder Bleichlaugen, beispielsweise in Toiletten- bzw. WC-Reinigern bzw. -steinen und beispielsweise auch in Abfluß- bzw. Rohrreinigern sowie z.B. auch in Grill- oder Backofenreinigern oder in sonstigen Metallreinigern eingesetzt werden kann.

Die vorliegende Erfindung betrifft daher in einem weiteren Aspekt auch Verbrauchs- oder Gebrauchsgüter oder Mittel, umfassend eine organoleptisch wirksame Menge der erfindungsgemäßen Gemische, umfassend 3,7-Dimethyl-2,6-nonadiennitril und mindestens eine Verbindung ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt.

Es wurde darüber hinaus gefunden, dass sich die erfindungsgemäßen Gemische umfassend 3,7-Dimethyl-2,6-nonadiennitril und mindestens eine Verbindung ausgewählt aus der Gruppe der Verbindungen 3,7-Dimethyl-3,6-nonadien-nitril und 3-Methylen-7-Methyl-6-nonennitril, wobei der Gehalt an 3,7-Dimethyl-3,6-nonadiennitril und 3-Methylen-7-methyl-6-nonennitrü zusammen bezogen auf die Menge an 3,7-Dimethyl-2,6-nonadiennitril im Gemisch 0,05 bis 6 Gew.-% beträgt, in besonderem Maße eignen zur Abmischung mit den Riechstoffen Citronellylnitril (3,7-Dimethyl-6-octennitril) der Formel (VII) 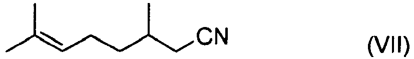 und/oder Dihydrocitronellylnitril (3,7-Dimethyloctannitril) der Formel (VIII), 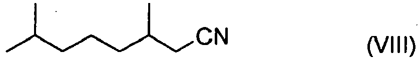 wobei generell beliebige Mischungsverhältnisse zur Anwendung gelangen können.

Die vorliegende Erfindung betrifft daher in einem weiteren Aspekt Duftstoffkompositionen nach Anspruch 5 sowie mindestens eine weitere Komponente ausgewählt aus den Duftstoffen Dihydrocitronellylnitril (3,7-Dimethyloctannitril) und Citronellylnitril (3,7-Dimethyl-6-octennitril). Derartige Duftstoffkompositionen eigenen sich insbesondere zur Substitution des Riechstoffes Geranonitril . Die erfindungsgemäßen Duftstoffkompositionen enthalten demnach ein Gemisch nach Anspruch 5 und wahlweise Citronellylnitril oder Dihydrocitronellylnitril, bevorzugt Citronellylnitril und Dihydrocitronellylnitril oder besonders bevorzugt nur Citronellylnitril.

Im Rahmen dieses Aspekts der vorliegenden Erfindung insbesondere bevorzugte Duftstoffkompositionen sind solche, die, jeweils bezogen auf das Gesamtgewicht der fertigen Duftstoffkomposition, 1 oder bevorzugt 2 bis 20 Gew.-%, bevorzugt 5 bis 10 Gew.-% Dihydrocitronellylnitril, 20 bis 60 Gew.-%, bevorzugt 35 bis 45 Gew.-% Citronellylnitril und 35 bis 75 Gew.-%, bevorzugt 50 bis 60 Gew.-% eines Gemischs aus 3,7-Dimethyl-2,6-nonadiennitril und mindestens einer Verbindung ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt, enthalten. Derartige Duftstoffkompositionen weisen bis hin zu feinen Nuancen Analogien zum Geruchseindruck des Geranonitrils auf und können in gleicher Weise sowie in etwa gleicher Dosierung wie das zu substituierende Geranonitril eingesetzt werden.

Die erfindungsgemäßen Duftstoffkompositionen können mit den auf diesem Anwendungsgebiet üblichen Lösemitteln beliebig verdünnt werden. Beispielhaft seien als geeignete Lösemittel genannt Ethanol, Dipropylenglycol oder dessen Ether, Phthalate, Propylenglykole, oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die erfindungsgemäße Duftstoffkomposition zeichnet sich dadurch aus, dass sie Ethylgeranonitril in Form eines Gemisches, umfassend 3,7-Dimethyl-2,6-nonadiennitril und mindestens eine Verbindung ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadien-nitril im Gemisch 0,05 bis 6 Gew.-% beträgt, enthält.

Derartige Gemische sowie ihre bevorzugten Ausführungsformen, wie sie vorstehend im Rahmen der vorliegenden Erfindung beschrieben sind, eignen sich in besonderem Maße zur Einarbeitung in die erfindungsgemäßen Duftstoffkompositionen.

Die erfindungsgemäßen Duftstoffkompositionen weisen auf Grund der strukturellen und chemischen Ähnlichkeit der Komponenten eine hohe Stabilität und Haltbarkeit auf, zeichnen sich durch einen frischen zitronenartigen Geruch aus und stellen somit ebenfalls geeignete Substitute für den Riechstoff Geranonitril dar. Dabei ist als vorteilhaft hervorzuheben das die beiden möglichen Komponenten der Duftstoffkomposition Citronellylnitril und Dihydrocitronellylnitril jeweils wohlfeile und in technischem Maßstab gut zugängliche Verbindungen darstellen und somit dazu beitragen, den Bedarf an erfindungsgemäß zugänglichem Ethylgeranonitril bzw. der erfindungsgemäßen Gemische reduzieren zu können. Die vorliegende Erfindung betrifft daher in einem weiteren Aspekt die Verwendung der erfindungsgemäßen Duftstoffkompositionen zur Erzeugung eines Zitrusgeruchs.

Die erfindungsgemäßen Duftstoffkompositionen eigenen sich wie die vorstehend genannten Gemische ebenfalls zur Einarbeitung in Gebrauchs- und Verbrauchsgüter bzw. Mittel wie sie beispielhaft vorstehend für die genannten Gemische beschrieben sind. Ein weiterer Aspekt der vorliegende Erfindung betrifft daher Verbrauchs- oder Gebrauchsgüter die eine organoleptisch wirksame Menge der erfindungsgemäßen Duftstoffkompositionen umfassen, wobei die Duftstoffkomposition in die genannten Güter eingearbeitet oder auch auf solche aufgebracht sein kann. Unter einer organoleptisch wirksamen Menge ist dabei wie im Rahmen der gesamten vorliegenden Erfindung insbesondere eine solche Menge zu verstehen, die bei sachgemäßer Anwendung ausreicht beim Anwender bzw. Verbraucher einen Dufteindruck, speziell den Eindruck eines intensiven Zitrusgeruchs hervorzurufen.

Die folgenden Beispiele dienen der Erläuterung der Erfindung, ohne sie in irgendeiner Weise zu beschränken:

### Beispiel 1:

180 g (1 mol) Natriummethylat (30%-ige Lösung in Methanol) wurden in 410 g (10 mol) Acetonitril vorgelegt und auf 80°C erhitzt. Innerhalb von 1 h wurden 140 g (1 mol) Ethylheptenon in 200 ml Acetonitril zugetropft und das Reaktionsgemisch über Nacht unter Rückfluss erhitzt. Anschließend wurden 60 g (1 mol) Essigsäure zugetropft und das Gemisch mit 200 ml Wasser versetzt. Die Phasen wurden getrennt und die wässrige Phase dreimal mit je 100 ml Toluol extrahiert. Die organischen Phasen wurden vereinigt und das Lösungsmittel im Vakuum entfernt. Man erhielt 159 g eines Rohproduktes, welches gaschromatographisch analysiert wurde.

Das Rohprodukt wies die in Tabelle 4 angegebene Zusammensetzung auf (alle Angaben in GC-Flächen-%), wobei der Gehalt an den jeweils möglichen, nicht zugeordneten Konfigurations (E/Z-)-Isomeren bezüglich der jeweiligen ethylenischen Doppelbindung(en) angegeben wurde.

**Tabelle 1:**

| Produkt (GC FI%) | 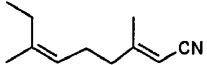 | | | | 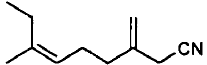 | | 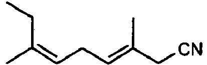 | | | |
|---|---|---|---|---|---|---|---|---|---|---|
| 80,6% | 12,4% | 26,8% | 19,1% | 40,7% | 0% | 0% | NB | 0,2% | 0,2% | 0,5% |

Das so erhaltene Rohprodukt wurde bei einer Sumpftemperatur von 120 bis 190°C und einem absoluten Druck von 3 bis 5 mbar fraktioniert destilliert. Man erhielt 92 g (0,56 mol entspr. 56% d.Th.) Ethylgeranonitril in Form eines Gemisches von Doppelbindungsisomeren.

### Beispiel 2:

224 g (4 mol) KOH-Plätzchen wurden in 580 g (14 mol) Acetonitril vorgelegt und auf 80°C erhitzt. Innerhalb von 2 h wurden 561 g (4 mol) Ethylheptenon zugetropft und das Reaktionsgemisch 4 h zum Rückfluss erhitzt. Das Reaktionsgemisch wurde auf Raumtemperatur abgekühlt und anschließend mit 600 ml Wasser und 300 ml Toluol versetzt. Die Phasen wurden getrennt und die organische Phase dreimal mit je 500 ml 25%-iger Schwefelsäure-Lösung gewaschen. Die organische Phase wurde über Natriumsulfat getrocknet und das Lösungsmittel im Vakuum entfernt. Man erhielt 674 g eines Rohproduktes, welches gaschromatographisch analysiert wurde.

Das Rohprodukt wies die in Tabelle 2 angegebene Zusammensetzung auf (alle Angaben in GC-Flächen-%), wobei der Gehalt an den jeweils möglichen, nicht zugeordneten Konfigurations (E/Z-)-Isomeren bezüglich der jeweiligen ethylenischen Doppelbindung(en) angegeben wurde.

**Tabelle 2:**

| Produkt (GC FI%) | 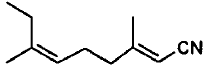 | | | | 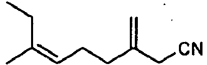 | | 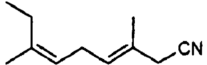 | | | |
|---|---|---|---|---|---|---|---|---|---|---|
| 68,2% | 14,4% | 23,5% | 23,0% | 36,8% | 0% | 0% | NB | 0,1% | 0,2% | 0,3% |

Das so erhaltene Rohprodukt wurde zunächst mittels Sambayverdampfer bei einer Temperatur von 140 bis150°C und einem absoluten Druck von 5 mbar von Hochsiedem befreit und anschließend bei einer Sumpftemperatur von 120 bis 160°C und einem absoluten Druck von 10 mbar fraktioniert destilliert. Man erhielt 352 g (2,16 mol entspr. 54% d.Th.) Ethylgeranonitril in Form eines Gemisches von Doppelbindungsisomeren.

## Patentansprüche

1. Verfahren zur Herstellung eines Gemisches umfassend 3,7-Dimethyl-2,6-nonadiennitril und mindestens eine Verbindung, ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3,7-Dimethyl-2,6-nonadiennitril im Gemisch 0,05 bis 6 Gew.-% beträgt, umfassend die Schritte
a) Umsetzung eines Nitrils der Formel (II) 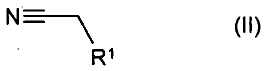 in dem der Rest
R¹ Wasserstoff bedeutet,
mit einer Base als Aktivierungsreagenz unter Erhalt des Nitrils der Formel (II) in deprotonierter Form und
b) Umsetzung des Nitrils der Formel (II) in deprotonierter Form mit 6-Methyl-5-octen-2-on unter Erhalt von 3,7-Dimethyl-2,6-nonadiennitril.

2. Verfahren nach Anspruch 1, **dadurch gekennzeichnet, dass** man es in Acetonitril als Lösungsmittel durchführt.

3. Verfahren nach Anspruch 1 oder 2, **dadurch gekennzeichnet, dass** man eine Base mit einem pKs-Wert von 12 bis 50 einsetzt.

4. Verfahren nach einem der Ansprüche 1 bis 3, **dadurch gekennzeichnet, dass** man das gemäß Schritt a) erhaltene Reaktionsgemisch vorlegt und 6-Methyl-5-octen-2-on zudosiert.

5. Gemisch, umfassend 3,7-Dimethyl-2,6-nonadiennitril und mindestens eine Verbindung, ausgewählt aus der Gruppe der Verbindungen 3,7-Dimethyl-3,6-nonadiennitril und 3-Methylen-7-methyl-6-nonennitril, wobei der Gehalt an 3,7-Dimethyl-3,6-nonadiennitril und 3-Methylen-7-methyl-6-nonennitril zusammen bezogen auf die Menge an 3;7-Dimethyl-2,6-nonadiennitril im Gemisch 0,05 bis 6 Gew.-% beträgt.

6. Gemisch nach Anspruch 5, **dadurch gekennzeichnet, dass** es zu mindestens 95 Gew.-% aus den Verbindungen 3,7-Dimethyl-2,6-nonadiennitril, 3,7-Dimethyl-3,6-nonadiennitril und/oder 3-Methylen-7-methyl-6-nonennitril besteht.

7. Verwendung von Gemischen gemäß Anspruch 5 oder 6 als Riechstoffe.

8. Verwendung nach Anspruch 7 zur Erzeugung eines Zitrusgeruchs.

9. Verbrauchs- oder Gebrauchsgüter, umfassend eine organoleptisch wirksame Menge der Gemische gemäß einem der Ansprüche 5 oder 6.

10. Duftstoffkomposition, enthaltend ein Gemisch nach Anspruch 5 sowie mindestens eine weitere Komponente, ausgewählt aus den Duftstoffen 3,7-Dimethyloctannitril und 3,7-Dimethyl-6-octennitril.

11. Duftstoffkomposition nach Anspruch 10, enthaltend, jeweils bezogen auf das Gesamtgewicht der fertigen Duftstoffkomposition, 5 bis 10 Gew.-% 3,7-Dimethyloctannitril, 35 bis 45 Gew.-% 3,7-Dimethyl-6-octennitril und 50 bis 60 Gew.-% 3,7-Dimethyl-2,6-nonadiennitril.

12. Duftstoffkomposition nach Anspruch 10 oder 11, enthaltend 3,7-Dimethyl-2,6-nonadiennitril in Form eines Gemisches nach einem der Ansprüche 5 oder 6.

13. Verwendung einer Duftstoffkomposition nach einem der Ansprüche 10 bis 12 zur Erzeugung eines Zitrusgeruchs.

14. Verbrauchs- oder Gebrauchsgüter, umfassend eine organoleptisch wirksame Menge der Duftstoffkompositionen gemäß einem der Ansprüche 10 bis 12.

## Claims

1. A method of producing a mixture comprising 3,7-dimethyl-2,6-nonadienenitrile and at least one compound selected from the group of the compounds 3,7-dimethyl-3,6-nonadienenitrile and 3-methylene-7-methyl-6-nonenenitrile, where the content of 3,7-dimethyl-3,6-nonadienenitrile and 3-methylene-7-methyl-6-nonenenitrile together, based on the amount of 3,7-dimethyl-2,6-nonadienenitrile in the mixture, is from 0.05 to 6% by weight, comprising the steps
a) reaction of a nitrile of the formula (II) 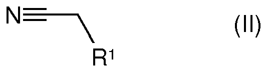 in which the radical
R¹ is hydrogen,
with a base as activation reagent to give the nitrile of the formula (II) in deprotonated form and
b) reaction of the nitrile of the formula (II) in deprotonated form with 6-methyl-5-octen-2-one to give 3,7-dimethyl-2,6-nonadienenitrile.

2. The method according to claim 1, which is carried out in acetonitrile as solvent.

3. The method according to claim 1 or 2, wherein a base with a pKa value of from 12 to 50 is used.

4. The method according to one of claims 1 to 3,
wherein the reaction mixture obtained according to step a) is initially introduced and 6-methyl-5-octen-2-one is metered in.

5. A mixture comprising 3,7-dimethyl-2,6-nonadienenitrile and at least one compound selected from the group of the compounds 3,7-dimethyl-3,6-nonadienenitrile and 3-methylene-7-methyl-6-nonenenitrile, where the content of 3,7-dimethyl-3,6-nonadienenitrile and 3-methylene-7-methyl-6-nonenenitrile together, based on the amount of 3,7-dimethyl-2,6-nonadienenitrile in the mixture, is from 0.05 to 6% by weight.

6. The mixture according to claim 5 which consists, to at least 95%, of the compounds 3,7-dimethyl-2,6-nonadienenitrile, 3,7-dimethyl-3,6-nonadienenitrile and/or 3-methylene-7-methyl-6-nonenenitrile.

7. The use of mixtures according to claim 5 or 6 as fragrances.

8. The use according to claim 7 for producing a citrus odor.

9. A consumer article or everyday commodity comprising an organoleptically effective amount of the mixtures according to either of claims 5 or 6.

10. A fragrance composition comprising a mixture
according to claim 5 and at least one further component selected from the fragrances 3,7-dimethyloctanenitrile and 3,7-dimethyl-6-octenenitrile.

11. The fragrance composition according to claim 10 comprising, in each case based on the total weight of the finished fragrance composition, 5 to 10% by weight of 3,7-dimethyloctanenitrile, 35 to 45% by weight of 3,7-dimethyl-6-octenenitrile and 50 to 60% by weight of 3,7-dimethyl-2,6-nonadienenitrile.

12. she fragrance composition according to claim 10 or 11 comprising 3,7-dimethyl-2,6-nonadienenitrile in the form of a mixture according to either of claims 5 or 6.

13. The use of a fragrance composition according to any of claims 10 to 12 for producing a citrus odor.

14. A consumer article or everyday commodity
comprising an organoleptically effective amount of the fragrance compositions according to any of claims 10 to 12.

## Revendications

1. Procédé pour la préparation d'un mélange comprenant du 3,7-diméthyl-2,6-nonadiènenitrile et au moins un composé, choisi dans le groupe des composés 3,7-diméthyl-3,6-nonadiènenitrile et 3-méthylène-7-méthyl-6-nonènenitrile, la teneur en 3,7-diméthyl-3,6-nonadiènenitrile et en 3-méthylène-7-méthyl-6-nonènenitrile ensemble, par rapport à la quantité de 3,7-diméthyl-2,6-nonadiènenitrile dans le mélange, valant 0,05 à 6% en poids, comprenant les étapes
a) transformation d'un nitrile de formule (II) 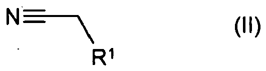 dans lequel le radical
R¹ signifie hydrogène, avec une base comme réactif d'activation avec obtention du nitrile de formule (II) sous forme déprotonée et
b) transformation du nitrile de formule (II) sous forme déprotonée avec de la 6-méthyl-5-octén-2-one avec obtention de 3,7-diméthyl-2,6-nonadiènenitrile.

2. Procédé selon la revendication 1, **caractérisé en ce qu'**on le réalise dans de l'acétonitrile comme solvant.

3. Procédé selon la revendication 1 ou 2, **caractérisé en ce qu'**on utilise une base présentant une valeur pKa de 12 à 50.

4. Procédé selon l'une quelconque des revendications 1 à 3, **caractérisé en ce qu'**on dispose au préalable le mélange réactionnel obtenu selon l'étape a) et on ajoute en dosant la 6-méthyl-5-octén-2-one.

5. Mélange, comprenant du 3,7-diméthyl-2,6-nonadiènenitrile et au moins un composé, choisi dans le groupe des composés 3,7-diméthyl-3,6-nonadiènenitrile et 3-méthylène-7-méthyl-6-nonènenitrile, la teneur en 3,7-diméthyl-3,6-nonadiènenitrile et en 3-méthylène-7-méthyl-6-nonènenitrile ensemble, par rapport à la quantité de 3,7-diméthyl-2,6-nonadiènenitrile dans le mélange, valant 0,05 à 6% en poids.

6. Mélange selon la revendication 5, **caractérisé en ce qu'**il est constitué à raison d'au moins 95% en poids des composés 3,7-diméthyl-2,6-nonadiènenitrile, 3,7-diméthyl-3,6-nonadiènenitrile et/ou 3-méthylène-7-méthyl-6-nonènenitrile.

7. Utilisation de mélanges selon la revendication 5 ou 6 comme substances odoriférantes.

8. Utilisation selon la revendication 7 pour générer une odeur d'agrume.

9. Objets ou biens de consommation, comprenant une quantité organoleptiquement active des mélanges selon l'une quelconque des revendications 5 ou 6.

10. Composition parfumée, contenant un mélange selon la revendication 5 ainsi qu'au moins un autre composant, choisi parmi les parfums 3,7-diméthyloctanenitrile et 3,7-diméthyl-6-octènenitrile.

11. Composition parfumée selon la revendication 10, contenant, à chaque fois par rapport au poids total de la composition parfumée finie, 5 à 10% en poids de 3,7-diméthyloctanenitrile, 35 à 45% en poids de 3,7-diméthyl-6-octènenitrile et 50 à 60% en poids de 3,7-diméthyl-2,6-nonadiènenitrile.

12. Composition parfumée selon la revendication 10 ou 11, contenant du 3,7-diméthyl-2,6-nonadiènenitrile sous forme d'un mélange selon l'une quelconque des revendications 5 ou 6.

13. Utilisation d'une composition parfumée selon l'une quelconque des revendications 10 à 12 pour générer une odeur d'agrume.

14. Objets ou biens de consommation, comprenant une quantité organoleptiquement active des compositions parfumées selon l'une quelconque des revendications 10 à 12.
